# EUROPÄISCHE PATENTSCHRIFT

(11) **EP 1 523 477 B1**
(45) Veröffentlichungstag und Bekanntmachung des Hinweises auf die Patenterteilung: **02.11.2005**
(21) Anmeldenummer: 03763731.1
(22) Anmeldetag: 07.07.2003
(51) Int. Cl.: C07D 307/62

(54) **VERFAHREN ZUR ABTRENNUNG VON ASCORBINSÄURE AUS EINEM POLAREN, ASCORBINSÄURE UND 2-KETO-L-GULONSÄURE ENTHALTENDEN LÖSUNGSMITTEL**
METHOD FOR THE SEPARATION OF ASCORBIC ACID FROM A POLAR SOLVENT CONTAINING ASCORBIC ACID AND 2-KETO-L-GULONIC ACID
PROCEDE DE SEPARATION D'ACIDE ASCORBIQUE A PARTIR D'UN SOLVANT POLAIRE CONTENANT DE L'ACIDE ASCORBIQUE ET DE L'ACIDE 2-CETO-L-GULONIQUE

(30) Priorität: 12.07.2002 DE 10231890
(43) Veröffentlichungstag der Anmeldung: 20.04.2005
(73) Patentinhaber: BASF Aktiengesellschaft, 67056 Ludwigshafen (DE)
(72) Erfinder: KAIBEL, Gerd, 68623 Lampertheim (DE); MERGER, Martin, 67061 Ludwigshafen (DE); DOMSCHKE, Thomas, 67346 Speyer (DE); DECKERT, Petra, 69168 Wiesloch (DE); SAUER, Friedrich, 67271 Obersülzen (DE)
(86) Internationale Anmeldenummer: PCT/EP2003/007256
(87) Internationale Veröffentlichungsnummer: WO 2004/007474

(56) Entgegenhaltungen:
- CH-A- 187 933
- US-A- 5 041 563
- US-B1- 6 197 977

## Beschreibung

### Zusammenfassung

Die vorliegende Erfindung betrifft ein Verfahren zur Abtrennung von Ascorbinsäure aus einem Gemisch enthaltend Ascorbinsäure und 2-Keto-L-gulonsäure in einem polaren, vorzugsweise wässrigen Lösungsmittel mittels Flüssig-Flüssig-Extraktion mit einem Amid. Vorzugsweise umfasst das erfindungsgemäße Verfahren auch Schritte zum Rückextrahieren der Ascorbinsäure, Rückführen des Extraktionsmittels und/oder des Rückextraktionsmittels und zur Isolierung der Ascorbinsäure aus dem Rückextraktionsmittel. Die vorliegende Erfindung betrifft ebenfalls ein Verfahren zur Herstellung von Ascorbinsäure aus KGS und Isolierung der hergestellten Ascorbinsäure.

### Beschreibung

L-Ascorbinsäure (Vitamin C, Ascorbinsäure, L-xylo-Ascorbinsäure, L-threo-hex-2-enolsäure-τ-lakton) wird üblicherweise aus 2-Keto-L-gulonsäure (KGS), Monoaceton-2-keto-L-gulonsäure oder Diaceton-ketogulonsäure hergestellt. In neueren Verfahren wird KGS in einem ein- oder mehrstufigen fermentativen Prozess gewonnen, beispielsweise durch die zweistufige Fermentation von Sorbitol über Sorbose mit hierzu geeigneten, teils speziell modifizierten Mikroorganismen.

KGS bzw. die im "Reichstein-Verfahren" anfallende Diaceton-2-keto-L-gulonsäure wird direkt oder über zwischenstufen wie z.B. Ester, insbesondere Methyl- oder Butylester, lactonisiert. Als Katalysator werden Säuren, meist Mineralsäuren, insbesondere konzentrierte Salzsäure (saure Lactonisierung) oder Basen wie z.B. Natronlauge, NaHCO₃, Na₂CO₃, Alkoholate usw. (alkalische Lactonisierung) eingesetzt. Ebenso ist die autokatalytische Umsetzung von KGS zu Ascorbinsäure beschrieben. Als Produkt der Lactonisierungsreaktion entsteht Rohascorbinsäure mit einem mehr oder weniger hohen Anteil an KGS, woraus dann die Ascorbinsäure aufgereinigt wird.

Ascorbinsäure und KGS unterscheiden sich im wesentlichen in ihrer chemischen Struktur nur durch die während der Lactonisierung gebildete Lactonstruktur der Ascorbinsäure. Dementsprechend ähneln sie sich in ihren chemischen Reaktionseigenschaften und haben ähnliche physikalische Eigenschaften. So zeigen beide Säuren unter den üblichen verfahrenstechnischen Herstellung- und Aufreinigungsbedingungen eine pH- und temmeraturabhängige Neigung zu Zersetzung und Bildung gefärbter Nebenkcaponenten. Die Löslichkeit der KGS und Ascorbinsäure wird durch die vier hydrophilen Hydroxy-Gruppen und die Säure-Gruppe geprägt. Beide besitzen ein ähnliches Löslichkeitsprodukt: sie sind gut in polaren Lösungsmitteln, insbesondere Wasser, aber nur wenig in unpolaren organischen Medien löslich.

Eine wirtschaftliche Auftrennung eines Gemisches aus Ascorbinsäure und RGS ist daher schwierig. Dies zeigt sich insbesondere bei den im Stand der Technik für die Ascorbinsäureherstellung beschriebenen Verfahrensschritten für die Trennung der Ascorbinsäure vom, nicht umgesetzten Edukt KGS oder seinen Derivaten.

Gemäß JP 85019285 können Ascorbinsäure und KGS aus wässriger Lösung durch Kristallisation der KGS als Na-KGS voneinander abgetrennt werden. Na-KGS muss in einem Folgeschritt wieder in die freie KGS überführt werden.

Bei alkalisch katalysierten Verfahren entsteht zunächst das Natriumsalz der Ascorbinsäure, das in einem weiteren Verfahrensschritt in die freie ACS überführt werden muss und mit einem äquimolaren Anfall an NaCl oder Na₂SO₄ verbunden ist. Danach ist meist ein weiterer Kristallisationsschritt nötig.

Ein Verfahren, welches freie Ascorbinsäure ohne Salzanfall liefert, ist in US 5041563 beschrieben. Hier wird die basisch katalysierte Lactonisierung eines KGS-Esters mit einem langkettigen Amin in einem dipolaren Lösungsmittel zu einem Ammoniumascorbat vorgeschlagen. Die Ascorbinsäurefreisetzung wird dann durch Extraktion des Amins mit einem unpolaren Lösungsmittel herbeigeführt. Dabei werden auch farbige Nebenprodukte mitextrahiert.

Katalysatorfreie Methoden zur Ascorbinsäuresynthese aus KGS-Estern sind seit ca. 1940 bekannt. Die Lactonisierung von KGS-Estern zu Ascorbinsäure erfolgt durch einfaches Erhitzen in Wasser, Alkoholen oder Gemischen von Wasser mit einem hydrophilen Lösungsmittel bei Temperaturen oberhalb 130°C und Verweilzeiten von 30 Minuten bis 90 Stunden. Ein Zusatz von Zitronensäure und Phosphat als Puffer zum Einstellen eines konstanten pH-Wertes soll die Ausbeuten steigern können. Nachteilig ist auch hier, dass die Salze anschließend wieder abgetrennt werden müssen.

In DE 861 841 ist eine Direktlactonisierung eines KGS-Esters mit Partialumsatz und Ascorbinsäure-Abtrennung durch selektive Kristallisation und Eduktrackführung beschrieben. Nicht umgesetztes Edukt wird durch Kristallisation der Ascorbinsäure abgetrennt.

Das Edukt darf nach der Kristallisation nur in geringer Konzentration in der Mutterlauge vorliegen, da sonst das Produkt verunreinigt wird. Daher muss bei hohen Umsätzen operiert werden.

US 1,904,619 beschreibt ein Verfahren zur kontinuierlichen KGS (-Derivat)-Lactonisierung unter Partialumsatz in wässriger Lösung. Das Produkt wird durch Kristallisation und Umkristallisation aus Methanol isoliert. Alle Mutterlaugen müssen vereinigt, aufkonzentriert und wieder in eine wässrige Lösung überführt werden.

Bisher wurden im Stand der Technik keine wirtschaftlichen Verfahren zur Trennung von Ascorbinsäure und KGS zur Verfügung gestellt, sodass in der Regel in den Herstellungsprozessen für Ascorbinsäure die Lactonisierung unter Vollumsatz der KGS oder des jeweiligen Eduktes durchgeführt werden muss, um eine Verunreinigung der Ascorbinsäure mit KGS zu vermeiden. Die Herstellung von Ascorbinsäure zeichnet sich aber durch besondere Anforderungen an Reinheit und Ausbeute in allen Verfahrensstufen aus: zum einen, um dem Einsatz des Endprodukts bei der Anwendung zur menschlichen Ernährung zu ermöglichen und andererseits, um die Kosten bei der Herstellung möglichst zu verringern.

Bei vielen Verfahren wird das Edukt oder Produkt derivatisiert. So werden insbesondere die Methyl- oder Butyl-Ester der KGS hergestellt, die in Alkahol im Gegensatz zu Ascorbinsäure löslich sind. Die beschriebenen Trennverfahren sind insbesondere durch die Derivatisierungsschritte und folgenden Freisetzungsschritte sehr komplex, zeitaufwendig, wenig effizient und aufgrund des hohen Verbrauchs an Energie sowie der Verwendung von organischen, größtenteils toxischen Lösungsmitteln ökologisch bedenklich.

Die Aufgabe der vorliegend Erfindung ist es folglich, ein vorteilhaftes Verfahren zur Verfügung zu stellen, um Ascorbinsäure und 2-Keto-L-gulonsäure wirtschaftlich, ökologisch und effizient aus einem polaren, vorzugsweise wässrigen Lösungsmittel trennen zu können. Das der vorliegenden Erfindung zugrunde liegende Problem wird durch die in den Ansprüchen der vorliegenden Erfindung charakterisierten Ausführungsformen gelöst.

Die vorliegende Erfindung betrifft folglich ein Verfahren zur Abtrennung von Ascorbinsäure aus einem polaren, Ascorbinsäure und 2-Keto-L-gulonsäure enthaltenden Lösungsmittel 1, dadurch gekennzeichnet, dass das Verfahren folgenden Schritt umfasst:
(a) Extrahieren der Ascorbinsäure aus dem Lösungsmittel 1 mit Dialkylformamid (Extraktionsmittel 1), das mit dem Lösungsmittel 1 eine Mischungslücke aufweist, in einer Flüssig-Flüssig-Extraktion.

In DB 38 31 071 wird KGS in Gegenwart von zwei bis sechs Moläquivalenten eines langkettigen Amins bei einem CO₂-Partialdruck von 10 bis 60 bar extrahiert.

In EP 359 645 wird eine verdünnte Lösung von KGS mit einer volumengleichen Lösung eines Amins (Adogen 83) in Kerosin extrahiert und mit Salpetersäure rückextrahiert.

GB 1,426,018 beschreibt die Gewinnung von u.a. Zitronensäure, Milchsäure und Oxalsäure aus wässrigen Lösungen mittels Extraktion.

Darauf aufbauend offenbart EP 828 725 ein Verfahren zur Gewinnung von Ascorbinsäure durch Extraktion der Ascorbinsäure mit einer mit Wasser nicht mischbaren Zusammensetzung, die (a) mindestens ein sekundäres oder tertiäres Alkylamin, bei dem die Gesamtzahl der Kohlenstoffatome mindestens 20 beträgt, als primäres Extraktionsmittel und (b) eine polare Extraktionsverstärkerverbindung ("Enhancer") enthält.

Bisher wurde jedoch nicht gezeigt, dass sich die beiden ähnlichen organischen Carbonsäuren Ascorbinsäure und RGS durch eine Flüssig-Flüssig-Extraktion voneinander trennen lassen.

Überraschenderweise kann aus einem polaren Lösungsmittel, das sowohl KGS als auch Ascorbinsäure gelöst enthält, Ascorbinsäure selektiv durch die Extraktion mit einem Dialkylformamid in hoher Reinheit großtechnisch abgetrennt werden.

Unter dem Begriff "Extraktion" oder "Extrahieren" wird erfindungsgemäß verstanden, dass aus einer festen oder flüssigen probe mit unpolaren bis polaren Lösungsmitteln oder Lösungsmittelgemischen die darin enthaltenen Substanzen in das jeweilige Extraktionsmittel oder Extraktionsmittelgemisch überführt werden. Unter Extraktionsmittel wird im folgenden auch ein Gemisch aus verschiedenen Lösungsmitteln verstanden, solange das Gemisch die hierin für das Extraktionsmittel beschriebenen Eigenschaften aufweist.

Unter einer "Flüssig-Flüssig"-Extraktion wird erfindungsgemäß eine Extraktion eines in einem flüssigen Lösungsmittel gelösten Stoffes mittels eines zweiten flüssigen Lösungsmittels verstanden. Die Extraktionsbedingungen, z.B. das Extraktionsmittel oder die Temperatur, können so gewählt sein, dass eine spezifische Substanz im wesentlichen oder bevorzugt extrahiert oder nicht extrahiert wird.

Polare Lösungsmittel sind erfindungsgemäß wässrige Lösungen, inklusive Wasser, oder polare aprotische oder protische organische Lösungsmittel, bspw. Alkylalkohole mit einem Alkylrest mit 1 bis 4 Kohlenstoffatomen, z.B. Methanol, Ethanol, 1-Propanol, 2-Propanol, Butanol, oder z.B. Aceton, Acetonitril, oder Dimethylsulfoxid oder es sind Gemische davon.

Unter einer "polaren Phase" oder einem "polaren Extrakt" wird eine Phase oder ein Extrakt verstanden, die/der aus einer Extraktion mit einem oben beschriebenen polaren Lösungsmittel oder Lösungsmittelgemisch erhalten wird.

Unter dem Begriff "wässrige Lösung" wird Wasser oder eine wässrige Lösung verstanden, auch z.B. deionisiertes, demineralisiertes, destilliertes oder bidestilliertes Wasser. Es können eine oder mehr Substanzen in der wässrigen Lösung gelöst oder damit vermischt sein. So können Substanzen enthalten sein, die die Extraktion, Stabilität oder Löslichkeit der Wertstoffe verbessern oder zu bevorzugten Eigenschaften, z.B. pH-Wert, Leitfähigkeit, Salzkonzentration usw., führen, wie z.B. Salz- oder Pufferlösungen.

Unter "Extraktionsmittel 1" wird erfindungsgemäß ein Lösungsmittel oder Lösungsmittelgemisch verstanden, das nicht mit dem Lösungsmittel 1 mischbar ist und eine Mischungslücke mit dem Lösungsmittel 1 besitzt. Unter dem Begriff "Mischungslücke" wird hierin verstanden, das Ascorbinsäure und/oder KGS eine höhere Löslichkeit in dem Extraktionsmittel besitzen als in dem extrahierten Lösungsmittel.

Eine wirtschaftliche Trennung durch Extraktion ist dann möglich, wenn die Verteilungskoeffizienten der beiden zu trennenden Substanzen, hier Ascorbinsäure und KGS, in einem Extraktionsmittel ausreichend unterschiedlich ist. Aufgrund der strukturellen Ähnlichkeit von Ascorbinsäure und KGS war dies nicht zu erwarten. Dies zeigt sich auch darin, dass, obwohl die Vorteile einer partiellen autokatalytischen Lactonisierung, insbesondere der vorteilhafte Verzicht auf Katalysatoren, zwar seit 1940 bekannt sind, entsprechende Verfahren jedoch aufgrund des Fehlens geeigneter Trennverfahren für Edukt und Produkt großtechnisch nicht zum Einsatz kommen.

Die erfindungsgemäße Extraktion kann wie in den hierin zitierten Dokumenten oder wie in den Beispielen beschrieben durchgeführt werden, z.B. mittels einer Gegenstromextraktionskolonne oder einer Mischer-Dekanter-Kaskade (*Mixer-Settler*).

Vorzugsweise wird in dem erfindungsgemäßen Verfahren das Extraktionsmittel und das Lösungsmittel in einem Verhältnis von 1:1 bis 5:1 eingesetzt, bevorzugt ist ein Verhältnis von 2:1 bis 3:1.

In einer bevorzugten Ausführungsform ist das Extraktionsmittel ein N,N-Dialkylformamid, mit jeweils N-gebundenen C1- bis C5-Alkylresten. Besonders bevorzugt ist N,N-Dibutylformamid (DBF) als Extraktionsmittel. Überraschenderweise wurde festgestellt, dass insbesondere mit DBF selektiv Ascorbinsäure aus einem wässrigen KGS/Ascorbinsäure-Gemisch extrahiert werden kann.

Erfindungsgemäß ist eine wirtschaftliche Trennung von Ascorbinsäure aus einem Gemisch aus Ascorbinsäure und KGS erreichbar, wenn das Verhältnis der Verteilungskoeffizienten bei Normalbedingungen für Ascorbinsäure zu KGS mindestens 1,5:1, vorzugsweise 4:1, mehr bevorzugt 7:1 oder mehr ist, wobei der verteilungskoeffizient natürlich von der Temperatur abhängig ist. Der Verteilungskoeffizient kann nach dem Fachmann geläufigen Methoden bestimmt werden, z.B. nach einer einstufigen Extraktion mittels HPLC-Analyse und iodometrische Titration.

In einer bevorzugten Ausführungsform ist das erfindungsgemäße Verfahren dadurch gekennzeichnet, dass in der Flüssig-Flüssig-Extraktion kein Extraktions-"Enhancer" verwendet wird.

Überraschend wurde gefunden, dass im dem erfindungsgemäßen Verfahren für die Trennung von Ascorbinsäure und KGS im Unterschied zu dem im Stand der Technik beschriebenen Extraktionsverfahren (EP 828 725) kein Extraktions-"Enhancer" verwendet werden muss. EP 828 725 beschreibt die Extraktion der Ascorbinsäure aus einer wässrigen Lösung mit Hilfe eines "ersten Extraktionsmittels", das aus langkettigen Aminen besteht und einem Extraktions-"Enhancer", der aus einem polaren und protischen Extraktionsmittel besteht, wobei der "Echancer" ein schlechteres Extraktionsmittel ist als das erste Extraktionsmittel und wobei in der Extraktion ein Verhältnis von "Enhancer"/"erstem Extraktionsmittel" von 2:1 eingesetzt wird. Bevorzugte polare, insbesondere protische "Enhancer" sind laut EP 828 725 Alkanole, Ketone, Aldehyde, Ester und Ether von verschiedener Molekulargewichten. Unter dem Begriff "Extraktions-Enhancer" wird erfindungsgemäß somit die in EP 828 725 offenbarten polaren, insbesondere protischen Extraktionsmittel verstanden, insbesondere Alkanole, Ketone, Aldehyde, Ester und Ether von verschiedenen Molekulargewichten.

Unter dem Begriff "kein Extraktions-Enhancer" wird erfindungsgemäß verstanden, dass das Verhältnis eines Extraktions-Enhancers zu dem "Extraktionsmittel 1" 0:1 bis 1, 9:1, vorzugsweise 1:1, mehr bevorzugt 0,2:1, noch mehr bevorzugt 0, 05:1 ist. Am meisten bevorzugt ist der Anteil an dem Extraktions-"Enhancer" 0 Vol-% bis 1 Vol-% an dem Gesamtvolumen des Extraktionsmittels. In dem erfindungsgemäßen Verfahren können Ascorbinsäure und KGS ohne Zusatz eines solchen Enhancers getrennt werden.

In einer weiteren bevorzugten Ausführungsform wird in dem erfindungsgemäßen Verfahren das Extrahieren der Ascorbinsäure aus dem Lösungsmittel 1 mit C₁ bis C₅-N,N-Dialkylformamid als Extraktionsmittel 1 durchgeführt wird. So wird eine Phase 1, die mit Ascorbinsäure beladenes Extraktionsmittel 1 enthält, und eine Phase 2, die das Lösungsmittel 1 und KGS enthält, erhalten. Bevorzugt wird als Extraktionsmittel N,N-Dibutylformamid (DBF) eingesetzt.

Wie in den Beispielen gezeigt, eignet sich DBF als Extraktionsmittel 1 für Ascorbinsäure aus einem Gemisch aus Ascorbinsäure und KGS. Überraschenderweise ist das Verhältnis der Verteilungskoeffizienten von Ascorbinsäure und KGS in DBF 7:1.

In einer Ausführungsform wird in dem erfindungsgemäßen Verfahren als Lösungsmittel 1 eine wässrige Lösung, oder ein verzweigter oder unverzweigter C₁ bis C₄-Alkylalkohol, verwendet, vorzugsweise Wasser oder eine wässrige Lösung. Der Begriff "wässrige Lösungen" umfasst nach der hierin verwendeten Definition sowohl Wasser als auch Puffer, Fermentationslösungen, Salzlösungen und andere Lösungen, die Substanzen enthalten, um z.B. den pH, die Sterilität der Lösung oder die Stabilität der Substanzen zu beeinflussen.
Die Lösungsmittel 1 kann auch eine Fermentationsbrühe oder der Überstand einer dekantierten oder filtrierten Fermentationsbrühe sein.

In einer besonders bevorzugten Ausführungsform wird in dem erfindungsgemäßen Verfahren als Lösungsmittel 1 Wasser oder eine wässrige Lösung und als Extraktionsmittel 1 DBF verwendet.

Vorzugsweise findet in dem erfindungsgemäßen Verfahren die Extraktion bei einer Temperatur zwischen 10°C und 60°C statt. Besonders bevorzugt ist eine Temperatur zwischen 15°C und 30°C. Der Fachmann wird bei der Wahl der bevorzugten Temperatur die Extraktionseffizienz gegen den Rühlenergieeinsatz zum Erreichen der jeweiligen Temperaturen sowie gegen die Löslichkeit der Edukte bei den jeweiligen Extraktionstemperaturen abwägen. Aus ökonomischen und ökologischen Gründen kann eine Temperatur bevorzugt sein, die ohne zusätzliche Energiezufuhr zum Kühlen oder Erwärmen erreicht werden kann (Umgebungstemperatur). Um eine effiziente Rückextraktion zu ermöglichen kann eine höhere Temperatur gewählt werden. Am meisten bevorzugt ist die Durchführung des erfindungsgemäßen Verfahrensschritt bei 30°C bis 60°C, vorzugsweise bei 40°C.

In einer weiteren Ausführungsform umfasst das erfindungsgemäße Verfahren den folgenden weiteren Schritt:
(b) vollständiges oder partielles Rückextrahieren der Ascorbinsäure aus dem beladenen Extraktionsmittel 1 mit einem polaren Extraktionsmittel 2, wobei ein mit Ascorbinsäure beladenes Extraktionsmittel 2 erhalten wird.

Unter "vollständiges oder partielles Rückextrahieren" wird erfindungsgemäß verstanden, dass Ascorbinsäure im wesentlichen vorzugsweise zu 30 Gew.-% bis 100 Gew.-% in das Extraktionsmittel 2 rückextrahiert wird. Bevorzugt sind 50 Gew.-%, mehr bevorzugt sind 75 Gew.-% oder mehr.

Um eine effiziente Rückextraktion zu ermöglichen, ist die Konzentration der Ascorbinsäure vor der Rückextralction im Extraktionsmittel 2 geringer als in dem Extraktionsmittel, d.h. vorzugsweise beträgt der Anteil 5 Gew.-%, mehr bevorzugt 1 Gew.-% oder 0,1 Gew.-% oder geringer, am meisten bevorzugt sind 0 Gew.-%.

Das Extraktionsmittel 2 ist ein polares Lösungsmittel wie oben beschrieben, vorzugsweise ist es eine wässrige Lösung oder ein verzweigter oder unverzweigter C₁ bis C₄-Alkylalkohol.

Vorzugsweise wird in dem erfindungsgemäßen Verfahren das Extraktionsmittel 2 zu dem Extraktionsmittel 1 im Verhältnis 1:1 bis 5:1 eingesetzt, bevorzugt ist ein Verhältnis von 1:1 bis 3:1.

In einer bevorzugten Ausführungsform besteht das Extraktionsmittel 2 und das Lösungsmittel 1 im wesentlichen aus den gleichen Lösungsmittelkomponenten.

"Im wesentlichen aus den gleichen Lösungsmittelkomponenten bestehend" bedeutet hierin, dass die beiden Mittel im wesentlichen identisch sind und sich vorzugsweise in 30 % oder weniger ihrer Lösungsmittelbestandteile unterscheiden, mehr bevorzugt sind 10 %, noch mehr bevorzugt sind 5% oder weniger. So kann z.B. ein Mittel im wesentlichen aus einer wässrigen Lösung bestehen mit einem geringen Anteil an einem Alkylalkohol, während das andere Mittel nur aus einer wässrigen Lösung besteht. In einer bevorzugten Ausführungsform sind die beiden Mittel bezüglich ihrer Lösungsmittelkomponenten identisch. Bevorzugt ist das Extraktionsmittel 2 ebenfalls polar. Besonders bevorzugt handelt es sich bei Lösungsmittel 1 und Extraktionsmittel 2 um wässrige Lösungen.

In einer Ausführungsform bestehen Lösungsmittel 1 und Extraktionsmittel 2 aus den gleichen oder ähnlichen Lösungsmitteln, in denen im wesentlichen die gleichen Substanzen bis auf den Anteil an Ascorbinsäure und/oder KGS enthalten sind.

"Im wesentlichen die gleichen Substanzen bis auf den Anteil an Ascorbinsäure und/oder KGS enthalten" heißt, dass beide Mittel sich nur in 30 % der gelösten und ungelösten Bestandteile außer Ascorbinsäure und KGS unterscheiden, mehr bevorzugt sind 10 %, noch mehr bevorzugt sind 5% oder weniger.

In einer bevorzugten Ausführungsform ist in dem erfindungsgemäßen Verfahren die Extraktionstemperatur T₁ für die Extraktion der Ascorbinsäure aus dem Lösungsmittel 1, das ein Gemisch aus Ascorbinsäure und KGS enthält, niedriger als die Rückextraktionstemperatur T₂ für die Rückextraktion der Ascorbinsäure bzw. KGS aus dem Extraktionsmittel mit dem Extraktionsmittel 2. Bevorzugt ist eine Differenz von mehr als 5°C bis 100°C, mehr bevorzugt mehr als 15°C, noch mehr bevorzugt von 20°C.

Wie in der GB 1,426,018 gezeigt kann bei Rückextraktionen mit höheren Temperaturen als bei der Extraktion mit dem gleichen Lösungsmittel, z.B. Extraktion bei Raumtemperatur und Rückextraktion bei 100°C, in dem Rückextrakt eine hohe Konzentration erreicht werden, insbesondere eine Konzentration, die ähnlich zu der Ausgangskonzentration in dem Gemisch ist.

Folglich liegt in einer Ausführungsform der vorliegenden Erfindung die Temperatur der Extraktion bei 10°C bis 30°C und die der Rückextraktionstemperatur bei 20°C bis 80°C. Bevorzugt ist die Kombination von Umgebungstemperatur oder Raumtsnperatur, worunter hierin eine Temperatur von 15°C bis 30°C verstanden wird, mit einer Rückextraktionstenperatur von 40°C bis 60°C.

In einer Ausführungsform umfasst das erfindungsgemäße Verfahren auch den folgenden weiteren Schritt:
(c) Zurückführen des Extraktionsmittels 1, aus dem die Ascorbinsäure gemäß Schritt (b) rückextrahiert wurde, in die Extraktion nach Schritt (a).

Vorzugsweise wird das Extraktionsmittel 1 vor der Rückführung und Wiederverwendung als Extraktionsmittel 1 in Schritt (a) partiell oder vollständig ausgeschleust, aufgearbeitet und dann erst zurückgeführt. Durch die Ausschleusung werden Verunreinigungen entfernt. Die Reinigung des Extraktionsmittels kann z.B. durch Destillation, Micro- oder Nanofiltration oder Adsorption (z.B. an Aktivkohle) erfolgen.

Der Anteil an ausgeschleustem Material hängt im wesentlichen von der Reinheit des Lösungsmittels 1 und dem Anteil an rückextrahiertem Wertprodukt, d.h. an Ascorbinsäure in dem Extraktionsmittel nach erfolgter Rückextraktion ab. Enthält das Extraktionsmittel 1 nach der Rückextraktion mit Extraktionsmittel 2 nur geringe Anteile an Wertprodukt und einen hohen Anteil an Verunreinigungen, so kann ein großer Anteil an Extraktionsmittel ausgeschleust werden. Wird in der Rückextraktion nur partiell rückextrahiert, dann befindet sich in dem Extraktionsmittel noch ein hoher Anteil an Wertprodukt und der Fachmann wird routinemäßig den Verlust durch eine Ausschleusung gegen den Grad der Verunreinigung abwägen.

In einer Ausführungsform umfasst das Verfahren den Schritt
(d) Aufkonzentrieren des mit der Ascorbinsäure beladenen Extraktionsmittels 2;
   und optional den Schritt
(e) Rückführen des in (d) abgedampften Extraktionsmittels 2 (Brüden) in die Rückextraktion nach Schritt (b) als Extraktionsmittel 2.

Unter "Aufkonzentrieren" wird verstanden, dass die Probe in ihrem Volumen eingeengt wird und die Konzentration der zu konzentrierenden Substanz nach der Aufkonzentration höher als in der Ausgangslösung ist, ohne jedoch auszufallen. Bevorzugt wird bis an die Löslichkeitsgrenze der Ascorbinsäure aus dem beladenen Extraktionsmittel 2 abgezogen oder abgedampft. In einer bevorzugten Ausführungsform wird genau soviel Lösungsmittel abgedampft, dass sich in der kontinuierlichen Anlage mit Rückführungen stationare Zustände einstellen können. Vorzugsweise wird in Schritt (d) auf eine Ascorbinsäurekonzentration von 30 bis 50 % Ascorbinsäure bei 30°C bis 50°C aufkonzentriert.

Eine Aufkonzentration kann beispielsweise durch Erwärmen, insbesondere unter reduziertem Druck, beispielsweise in einem Umlaufverdampfer, Dünnschichtverdampfer etc. erfolgen. Durch Dialyse lassen sich ebenfalls Proben einengen. Die Einengung sollte schonend geschehen, vorzugsweise bei -20°C bis 100°C in Abhängigkeit von Reaktionsdauer, Druck und Lösungsmittel. Bevorzugt wird das Aufkonzentrieren bei 30°C bis 50°C, besonders bevorzugt unter reduziertem Druck durchgeführt. Je nach Lösungsmittel oder Lösungsmittelgemisch kann das Aufkonzentrieren bei Normaldruck (1013 mbar) bis 10 mbar durchgeführt werden. Bei wässrigen Lösungen wird vorzugsweise bei 500 mbar bis 50 mbar aufkonzentriert. In einer besonders bevorzugten Ausführungsform wird die Einengung einer Lösung bei 30°C bis 50°C, vorzugsweise bei 40°C, sowie 50 mbar bis 300 mbar, vorzugsweise bei 70 mbar, durchgeführt. Vorzugsweise wird die aufkonzentrierte Lösung nach jeder hierin beschriebenen Eindampfung auf Umgebungstemperatur oder auf 20 bis 25°C abgekühlt, z.B. mittels eines Wärmeaustauschers.

Das abgedampfte Lösungsmittel (Brüden) kann dann kondensieren und für die Rückextraktion in Schritt (b) wieder eingesetzt werden.

In einer Ausführungsform wird zur Gewinnung der Ascorbinsäure die Ascorbinsäure aus dem Extraktionsmittel 2 unmittelbar, oder nach Eindampfen und Abkühlen der Lösung, isoliert.

Folglich umfasst das erfindungsgemäße Verfahren in einer bevorzugten Ausführungsform auch den folgenden Schritt:
(f) Isolieren, vorzugsweise Kristallisieren, der Ascorbinsäure aus dem mit der Ascorbinsäure beladenen Extraktionsmittel 2, wobei eine Mutterlauge zurückbleibt.

Dem Fachmann sind verschiedene Verfahrensschritte zur Gewinnung der Ascorbinsäure aus polaren Lösungsmittel bekannt. So sind z.B. Verdampfungs-, Kühlungs- oder Verdrängungskristallisationen, aber auch verschiedene Trocknungsverfahren, z.B. Sprühtrocknung für Carbonsäuren, insbesondere auch für Ascorbinsäure, beschrieben. Zur Isolierung der Ascorbinsäure können auch unlösliche Salze oder Derivate gebildet werden, die dann in dem Lösungsmittel ausfallen. Bevorzugt wird die Ascorbinsäure durch eine Verdampfungs-, Kühlungs- und Verdrängungskristallisationen isoliert. Besonders bevorzugt wird in dem erfindungsgemäßen Verfahren die Ascorbinsäure durch Kühlkristallisation ausgefällt und als Feststoff isoliert.

Das erfindungsgemäße Verfahren umfasst in einer Ausführungsform den folgenden weiteren Schritt:
(g) Extrahieren von Ascorbinsäure aus der bei der Kristallisation der Ascorbinsäure gemäß Schritt (f) zurückbleibenden Mutterlauge.

Bevorzugt wird zum Rückextrahieren der Ascorbinsäure aus der Mutterlauge die Mutterlauge der Extraktion nach Schritt (a) zugeführt. Vorteilhafterweise wird die Mutterlauge auf die oberste (bzw. letzte) Stufe der ersten Extraktionskolonne (bzw. eines mehrstufigen Extraktionsapparates) geführt.

In einer weiteren Ausführungsform wird das mit KGS beladene Lösungsmittel 1, das nach der Extraktion gemäß Schritt (a) zurückbleibt, in einen Verfahrensschritt zur Herstellung von Ascorbinsäure aus KGS zurückgeführt (Schritt h) und z.B. mit neuer Feedlösung vermischt, die dann einer Lactonisierungsreaktion wie hierin beschrieben zugeführt wird.

Der Produktaustrag der Lactonisierungsreaktion kann dann zur Gewinnung von Ascorbinsäure den hierin beschriebenen erfindungsgemäßen Verfahrensschritten unterworfen werden. Vor der Extraktion der Ascorbinsäure nach Schritt (a) kann in einer Ausführungsform der Produktaustrag der Lactonisierungareaktion aufkonzentriert werden, wie z.B. oben beschrieben wurde. Nach der Aufkonzentration erfolgt vorteilhaft eine Abkühlung der Lösung und dann die Extraktion der Ascorbinsäure nach den oben beschriebenen Schritten.

Mit dem hierin beschriebenen erfindungsgemäßen Verfahren könnte auch Ascorbinsäure aus einem Gemisch aus Ascorbinsäure und KGS, Monoaceton-2-keto-L-gulonsäure und/oder Diacetonketogulonsäure oder anderen Derivaten der Ketogulonsäure abgetrennt werden.

Die vorliegende Erfindung betrifft in einer Ausführungsform auch ein Verfahren zur Herstellung von Ascorbinsäure aus 2-Keto-L-gulonsäure, das die folgenden Schritte enthält:
(aa) Partielles Lactonisieren von 2-Keto-L-gulonsäure zu Ascorbinsäure
(ab) Abtrennen der Ascorbinsäure aus dem Gemisch mit KGS nach dem erfindungsgemäßen Verfahren.

Das Gemisch aus KGS und Ascorbinsäure kann nach dem Fachmann bekannten Verfahren hergestellt werden, z.B. nach einem hierin beschriebenen Verfahren zur Lactonisierung von KGS oder deren Derivaten und ggf. weiterer Umsetzung. Bevorzugt wird das Gemisch durch eine direkte partielle Lactonisierung, insbesondere durch eine autokatalytische Lactonisierung von KGS zu Ascorbinsäure hergestellt.

Unter "partielle Lactonisierung" wird erfindungsgemäß eine nicht vollständige Umsetzung des Eduktes zu Ascorbinsäure verstanden. Vorzugsweise werden in dem erfindungsgemäßen Verfahren 10 Gew.-% bis 95 Gew.-% mehr bevorzugt 20 Gew.-% bis 50 Gew.-% des Eduktes zu Ascorbinsäure umgesetzt. Besonders bevorzugt ist eine Ausführungsform mit einem KGS-Partialumsatz von 20 Gew.-% bis 40 Gew.-%.

Die Lactonisierungsreaktion (aa) kann nach Verfahren, wie im Stand der Technik seit 1933 beschrieben, durchgeführt werden, solange eine Mischung aus dem Edukt, vorzugsweise KGS, und Ascorbinsäure in einem polaren Lösungsmittel, vorzugsweise in einer wässrigen Lösung, insbesondere Wasser, erhalten wird. Aufgrund der fehlenden Trennverfahren beschreibt die Literatur in der Regel Vollumsetzungen von KGS zu Ascorbinsäure oder verbindet bei nur partieller Umsetzung die Trermung mit der Derivatisierung der KGS zu einem Ester und folgender Kristallisation der Ascorbinsäure wie oben beschrieben.

Verfahren zu Lactonisierung sind in dem oben erwähnten Stand der Technik und den darin zitierten Dokumenten beschrieben, die hiermit ausdrücklich in den Offenbarungsgehalt dieser Beschreibung mit aufgenommen sind.

Das hierin beschriebene Verfahren könnte auch zur Trennung von Ascorbinsäure von anderen Ausgangsprodukten dienen. Ascorbinsäure wird üblicherweise aus 2-Keto-L-gulonsäure, Monoaceton-2-keto-L-gulonsäure oder Diacetonketogulonsäure hergestellt. Andere Edukte, wie z.B. L-Gulono-γ-lacton und das Natriumsalz des α-Alkyl-KGS-pyranosids wurden auch beschrieben.

Direktlactonisierungen werden meist sauer katalysiert, bevorzugt mit Salzsäure als Gas oder mit wässriger Salzsäure und sind im Stand der Technik lange bekannt.

DE 696 810 und DE 641 639 beschreiben die Lactonisierung über den Umweg der Bildung eines Esters der KGS bzw. der Diaceton-KGS-Ester. Der Ester ist in Alkoholen löslich ist, die ACS nicht, so dass die ACS als Feststoff ausfällt. Es können zusätzlich Halogenkohlenwasserstoffe als Fällungshilfsmittel zugeführt werden.

Bei alkalisch katalysierten Verfahren ist die Reaktionsgeschwindigkeit der Lactonisierung höher, was zu höheren Raum-Zeit-Ausbeuten in den Apparaten führt. Als basische Katalysatoren werden neben NaOH in verschiedenen Alkohol- oder Alkohol-Wasser-Mischungen, Alkalisalze schwacher Säuren (z.B. NaHCO₃ oder Natriumacetat), Na₂CO₃ oder Natriummethylat in Alkoholen eingesetzt. Bei diesen Verfahren entsteht zunächst das Natriumsalz der Ascorbinsäure, das in einem weiteren Verfahrensschritt in die freie Ascorbinsäure überführt werden muss. Ein Verfahren zur Herstellung von freier Ascorbinsäure ist in US 5,041,563 beschrieben.

Bei den genannten sauren Verfahren muss der Katalysator abgetrennt werden. Die Säure kann das Produkt zersetzen. Unter alkalischer Katalyse wird zunächst ein Ascorbinsäuresalz hergestellt, das in die freie Ascorbinsäure überführt werden muss.

Seid ca. 1940 ist auch die katalysatorfreie Lactonisierung von KGS und KGS-Estern zu Ascorbinsäure durch einfaches Erhitzen in Wasser, Alkoholen oder Gemischen von Wasser mit einem hydrophilen Lösungsmittel bei Temperaturen oberhalb 130°C und Verweilzeiten von 30 Minuten bis 90 Stunden beschrieben. Ein Zusatz von Zitronensäure und Phosphat als Puffer zum Einstellen eines konstanten pH-Wertes soll die Ausbeuten steigern können.

In DE 861 841 ist eine Direktlactonisierung mit Partialumsatz durch Erhitzen eines KGS-Esters in einem wasserhaltigen, mit Wasser mischbaren organischen Lösungsmittel (Alkohol/Wasser bzw. Ether/Wasser) und Produktabtrennung durch selektive Kristallisation und Eduktrückführung beschrieben. Das Edukt darf nach der Kristallisation jedoch nur in geringer Konzentration in der Mutterlauge vorliegen. US 2,491,065 beschreibt die autokatalytische Direktlactonisierung von KGS bzw. DAKS in wässriger Lösung bei einer KGS-Konzentration <12 Gew.-%, Temperaturen von 100-150°C und Verweilzeiten von 20 Minuten bis 10 Stunden- US 1,904,619 beschreibt ein Verfahren zur kontinuierlichen KGS (-Derivat)-Lactonisierung unter Partialumsatz in wässriger Lösung z.B. mit einem sauren Ionentauscher als Katalysator und Rückführung nicht umgesetzter KGS.

Vorteilhafterweise kann durch das erfindungsgemäße Verfahren nun eine direkte saure oder alkalisch katalysierte oder eine autokatalysierte Partiallactonisierung durchgeführt werden, z.B. durch einen sauren Ionenaustauscher (z.B. Bayer Lewatit) oder, bevorzugt, mittels einer Festbettkatalyse. Vorzugsweise wird die Lactonisierung bei geringen Temperaturen durchgeführt, die zu einer geringen Derivatisierung oder Zersetzung der entstandenen Ascorbinsäure führen, besonders bevorzugt unter 60°C, z.B. mittels Bio- oder Enzymkatalyse oder in einer sauren Katalyse.

In einer besonders bevorzugten Ausführungsform erfolgt in dem erfindungsgemäßen Verfahren der Schritt (aa) zur Lactonisierung der 2-Keto-L-gulonsäure autokatalytisch.

Die Lactonisierungen in den meisten Verfahren werden unter vollständigem Umsatz des jeweiligen Eduktes durchgeführt. Vorteilhaft bei der autokatalytischen Umsetzung ist, dass weder Katalysatoren noch sonstige Hilfsstoffe benötigt werden und aus dem Reaktionsaustrag abgetrennt werden müssen. Ein wirtschaftlicher Einsatz der autokatalytischen Lactonisierung scheiterte bisher daran, dass eine vollständige Umsetzung nicht effizient und mit geringen Ausbeuten erfolgt. Ein geeignetes Trennverfahren zur Gewinnung der Ascorbinsäure aus einem Gemisch aus RGS und Ascorbinsäure, wie es durch eine partielle Umsetzung erhalten wird, war nicht beschrieben und wird erst in der vorliegenden Erfindung zur Verfügung gestellt.

Es ist bekannt, dass KGS in wässrigen Lösungen durch Einwirkung einer erhöhten Temperatur (T > 25°C, T < 200°C) lactonisiert werden kann. Bevorzugt sind Temperaturen von 40 bis 180°C. Vorteilhaft kann so eine sehr kurze Umsetzungszeit im Reaktor erreicht werden. Erhitzt man eine Lösung von KGS in Wasser auf 80-150°C und hält die Verweilzeit im Reaktor zwischen 1 und 30min, können bei KGS-Dmsätzen um 25 - 30 % Ascorbinsäureselektivitäten um 90 % in Lösung erhalten werden. Partialumsatz mit Eduktrückfübrung wurde bisher nur im Falle der KGS-Ester beschrieben. Vorzugsweise überschreitet die Anfangskonzentration der KGS in Wasser 30 % nicht.

Bei einer besonders bevorzugten Ausführungsform wird eine Lactonisierung durchgeführt und dann Ascorbinsäure abgetrennt und das Edukt, insbesondere RGS, in die Lactonisierungsreaktion zurückgeführt.

Folglich betrifft die vorliegende Erfindung auch ein Verfahren zur Herstellung und Gewinnung von Ascorbinsäure, wobei der Schritt (aa) zur Lactonisierung der 2-Reto-L-gulonsäure autokatalytisch unter Partialumsatz unter folgenden Bedingungen durchgeführt wird:
(aaa) bei einer Temperatur von 60°C bis 180°C, vorzugsweise zwischen 100°C und 160°C;
(bbb) bei einem Anfangsmassenanteil der 2-Keto-L-gulonsäure von 5 Gew.-% bis 50 Gew.-%, vorzugsweise zwischen 10 % und 15 %;
(ccc) bei einer Umsetzung der KGS zu 10 bis 40 Gew.-%, vorzugsweise zu 20 bis 30 Gew.-%; und/oder
(ddd) einer Verweilzeit im Lactonisierungs-Reaktor von 1 bis 30 min, vorzugsweise 10 min oder weniger.

Besonders bevorzugt sind ein Anfangsmassenanteil der KGS von 10 bis 15 Gew.-%, eine Reaktortemperatur von 110°C bis 150°C bei einer Verweilzeit von 3 bis 5 min und eine Umsetzung an KGS von 20 bis 25 Gew.-%.

Als Reaktoren für die Lactonisierung eignen sich z.B. Rohrbündel, Plattenwärmetauscher, Wendelrohrreaktor oder Treibstrahlreaktoren.

Der Reaktionsaustrag aus der Lactonisierungsreaktion wird zur Erreichung eines stationären Betriebszustands gemäß den oben beschriebenen Aufkonzentrierungsschritten eingeengt. Aus dem vorzugsweise auf Umgebungstemperatur oder 20°C bis 25°C abgekühlten Reaktionsaustrag kann dann gemäß Schritt (a) die Ascorbinsäure oder die KGS abgetrennt werden.

Vorzugweise besitzt der Reaktionsaustrag nach der Aufkonzentrierung einen KGS-Anteil von 5 bis 30 Gew.-%, besonders bevorzugt sind 8 bis 25 Gew.-%, und einen Ascorbinsäureanteil von 3 bis 20 Gew.-%, besonders bevorzugt sind 5 bis 10 Gew.-%.

In einer bevorzugten Ausführungsform verbleiben in dem erfindungsgemäßen Verfahren die kondensierten Brüden der verschieden Eindampfungsschritte weitgehend im Prozess und werden dort als Lösungsmittel eingesetzt, wie es für die verschiedenen Verfahrensschritte oben beschrieben wurde. Besonders bevorzugt wird die Abdampfung der jeweiligen Lösungsmittel über den jeweiligen Betriebsdruck so betrieben, dass eine Energieübertragung vom Brüdenkondensator einer ersten Eindampfung auf den Verdampfer einer zweiten Eindampfung erfolgen kann, insbesondere von der Eindampfung nach der Lactonisierung zu der Eindampfung nach der Reextraktion der Ascorbinsäure (Schritt (d)).

Erfindungsgemäß können die einzelnen Schritte des hierin beschriebenen Verfahrens kontinuierlich oder diskontinuierlich ausgeführt werden. Bevorzugte Ausführungsform ist die kontinuierliche Ausführung der Schritte.

In einer Ausführungsform enthält das erfindungsgemäße Verfahren zur Gewinnung von Ascorbinsäure oder zur Herstellung von Ascorbinsäure alle hierin beschriebenen Schritte (a) bis (g) und/oder (aa) bis (cc) und/oder (aaa) bis (ccc). Vorteilhafterweise wird dadurch Ascorbinsäure und/oder KGS ohne Salzanfall gewonnen.

Die vorliegende Erfindung wird durch die folgenden Beispiele und Abbildungen verdeutlicht, ohne dass diese in irgendeiner Weise als einschränkend gellen.

In Abbildung 1 ist ein Verfahrensschema dargestellt.

In Abbildung 2 ist eine Laboranlage dargestellt. Die Ziffern weisen auf folgende Ausführungsformen hin:

| | |
|---|---|
| Lactonisierungsreaktor 1 | Wendelrohrreaktor |
| Eindampfung 2 | Labordünnschichtverdampfer |
| Wärmetauscher 3 | Doppelrohrwärmetauscher |
| Extraktionskolonne 4 | Gegenstromextraktionskolonne |
| Extraktionskolonne 5 | Gegenstromextraktionskolonne |
| Druckhalteventil 6 | Ventil |

### Beispiele:

### Beispiel 1: Verfahren

Zunächst wird eine Mischung aus Lösungsmittel (LM) und KGS (z.B. eine wäßrige Lösung einer Konzentration von 5 bis 50 % Massenanteil, insbesondere 10- 15 %), ohne weitere Hilfsstoffe mit einem indirekt oder direkt mit Dampf oder indirekt mit einem anderen Wärmeträger beheizten Reaktor (z.B. Rohrbündel-, Plattenwärmetauscher oder Treibstrahlreaktor) im Teilumsatz bei Temperaturen zwischen 80° und 180°C, insbesondere bei Temperaturen zwischen 100 und 150°C und Verweilzeiten von 1 -30 Minuten, insbesondere zwischen 1 und 10 Minuten lactonisiert. Hierbei wird der KGS-Anteil zu ca. 30 % mit 90 % Selektivität in ACS umgesetzt. Der Reaktoraustrag wird in einer Eindampfung 2, z.B. in einem Umlaufverdampfer, bei niederen Temperaturen (z.B. 40 - 80°C, und dem entsprechenden vakuum) aufkonzentriert und dann in einem Wärmetauscher 3 auf ca. 25°C abgekühlt. Der abgekühlte und aufkonzentrierte Reaktoraustrag enthält dann typischerweise KGS-Konzentrationen von 5-25 % (Massenanteil) und ACS-Konzentrationen von 1-10 % (Massenanteil). Im folgenden Verfahrensschritt, einer mehrstufigen Flüssig-Flüssig-Extraktion 4, wird die im Reaktionsaustrag enthaltende ACS mit einem geeigneten Extraktionsmittel (EM), z.B. N,N-Dibutylformamid (DBF) extrahiert. Hierbei wird das EM im Gegenstrom zum Reaktoraustrag, der in einer mittleren Stufe des mehrstufigen Apparates zugeführt wird, zugeführt. Ein geringer Strom an LM wird vorzugsweise auf die letzte (bzw. oberste) Stufe, ebenfalls im Gegenstrom, des mehrstufigen Extraktionsapparates zugegeben. Die Mutterlauge (ML) aus der Kristallisationsstufe 8 wird hierfür vorteilhaft benutzt. Reicht die Mutterlauge in ihrer Menge nicht aus, können zusätzlich kondensierte Brüden aus den Eindampfungen 2 und 6 zugeführt werden. Der LM-Austrag der mehrstufigen Extraktionsapparatur 4, der neben dem LM vorwiegend KGS enthält, wird in den Lactonisierungsreaktor 1 zurückgeführt. Der mehrstufige Extraktionsapparat kann z.B. eine sogenannte Mischer-Dekanter-Apparatur oder eine Extraktionskolonne sein. Das mit ACS beladene EM wird in einem zweiten mehrstufigen Extraktionsapparat 5 weitgehend von der ACS entladen. Als Extraktionsmittel, das wiederum im Gegenstrom geführt wird, können hier die kondensierten Brüden aus den Eindampfungen 2 und 6 benutzt werden. Die austretende Roh-ACS-Lösung wird in der Eindampfung 6 (z.B. bei ca. 40 - 60°C und entsprechendes Vakuum) auf eine ACS-Konzentration von ca. 45 % (m/m) eingedampft. Das entladene EM wird in die erste Extraktionsapparatur 4 zurückgeführt. Die Roh-ACS-Lösung wird anschließend mit Kühlwasser im Wärmetauscher 7 abgekühlt und anschließend dem Kristallisator 8 zugeführt. Die auskristallisierte Roh-ACS wird von der Mutterlauge abgetrennt (z.B. abzentrifugiert oder mit Bandfiltem abgetrenat). Die zurückbleibende Mutterlauge (ML, ca. 14 % ACS bei 2 °C Kristallisationstemperatur) wird wie bereits beschrieben auf die letzte (bzw. oberste) Stufe der ersten Extraktionsapparatur 4 zurückgeführt. Die Menge an zusätzlichem LM bzw. kondensierten Brüden wird durch die Trennstufenzahl des Apparates 4 und den tolerierbaren KGS-Schlupf bestimmt. Die Nebenprodukte der Lactonisierungsreaktion reichern sich überwiegend im EM-Kreislauf an, da die Nebenprodukte in der eher unpolaren EM-Phase besser löslich sind, als in der polaren LM-Phase. Zur Ausschleusung der hochsiedenden Nebenprodukte aus dem Prozeß wird ein Teilstrom des EM abgezogen, destilliert und wieder in den Prozeß zurückgeführt. Das Sumpfprodukt der EM-Aufarbeitung wird einer Rückstandsverbrennung oder einer biologischen Kläranlage zugeführt.

Die kondensierten Brüden aus den Eindampfungen 2 und 6 verbleiben bis auf einen kleinen Reststrom im Prozeß, das Verfahren ist also annähernd abwasserfrei.

Die bei der Kondensation der Brüden aus der ersten Eindampfung 2 freiwerdende Energie kann weitgehend für die zweite Eindampfung 6 genutzt werden, indem über die Wahl geeigneter Eindamgfungsdrücke eine ausreichende Temperaturdifferenz zwischen den Eindampfungen erzeugt wird.

### Beispiel 2: Laboranlage

Es wurde eine Laboranlage, gemäß Bild 2 aufgebaut. Die Anlage bestand aus den oben aufgeführten Teilen.

Die Anlage wurde wie folgt betrieben. In den Reaktor 1 wurde eine wässrige Lösung von KGS mit einer Konzentration von 9,83 % Massenanteil mit einem Massenstrom von 198 g/h und der Rückführstrom (Sumpfabzug der Extraktionskolonne 4) dosiert. Der Reaktor 1 war hierbei auf 160°C temperiert. Der Reaktor wurde durch ein Druckhalteventil 6 auf einem konstanten Druck von 10 bar gehalten. Der entspannte Reaktoraustrag enthielt eine ACS-Konzentration von 3,5 % und eine KGS-Konzentration von 6,7 %. Er wurde in den Dünnschichtverdampfer 2 überführt und dort bei 50°C und 150 mbar Druck teilweise verdampft. Der aufkonzentrierte Reaktoraustrag enthielt 5 % ACS und 9,43 % KGS. Die aufsteigenden Brüden wurden kondensiert. Vom Kondensat wurde soviel abgezogen (274 g/h), dass sich die Phasengrenzfläche zwischen der wässrigen Phase und der organischen Phase im oberen Teil der Kolonne 4 stabilisierte, der Rest wurde als Rücklauf wieder in den Verdampferkreislauf gegeben. Der aufkonzentrierte Reaktoraustrag wurde über eine Standregelung des Dünnschichtverdampfers, über den wassergekühlten Wärmetauscher 3 in die Extraktionskolonne 4 überführt. Der wässrige Sumpfabzug der Kolonne 4 wurde mit konstant 618 g/h abgezogen und in den Reaktor 1 zurückgeführt, er enthielt 2,5 % ACS und 8,73 % RGS. Am oberen Ende der Kolonneneinbauten wurden 95 g/h VE-Wasser zugeführt, am unteren Ende der Einbauten wurden 600 g/h wassergesättigtes N,N-Dibutylformamid aus der Extraktionskolonne 5 zugeführt. Das regenerierte Extraktionsmittel enthielt hier noch 0,58 % ACS. Der Kopfaustrag der Kolonne 4 enthielt ca. 3 % ACS. Er wurde am unteren Ende der Einbauten der Kolonne 5 eingebracht. Hier stellte sich ein Massenstrom von 619 g/h ein. Am oberen Ende der Kolonne 5 wurden 400 g/h VE-Wasser zugeführt. Der wässrige Sumpfaustrag der Kolonne 5 wurde so geregelt, daß sich die Phasengrenzschicht zwischen der wässrigen und der organischen Phase im oberen Teil der Kolonne stabilisierte. Es stellte sich ein Abzugsstrom von 416 g/h Rohproduktlösung mit einem ACS-Anteil von 2,9 % und einem KGS-Anteil von 0,47 % ein. Der Ropfaustrag der Kolonne 5 wurde, wie bereits beschrieben, am unteren Ende der Kolonneneinbauten wieder in die Kolonne 4 zurückgeführt. Vom umlaufenden Extraktionsmittel wurden am Kopf der Kolonne 5 ca. 10 % des Umlaufstromes als Purge-Strom zur Ausschleusung der Nebenprodukte abgezogen. Zur Aufrechterhaltung des Massenstromes an umlaufendem Extraktionsmittel wurde füllstandgeregelt frisches N,N-Dibutylformamid nachdosiert. Mit den genannten Konzentrationen und Mengenströmen ergibt sich eine ACS-Ausbeute von 68 %, die durch Verbesserung der Extraktion bzw. Rückführung der Kristallisationsmutterlauge in die Kolonne 4 auf 75 % gesteigert werden könnte.

## Patentansprüche

1. Verfahren zur Abtrennung von Ascorbinsäure aus einem polaren, Ascorbinsäure und 2-Keto-L-gulonsäure enthaltenden Lösungsmittel 1, **dadurch gekennzeichnet, dass** das Verfahren folgenden Schritt umfasst:
(a) Extrahieren der Ascorbinsäure aus dem Lösungsmittel 1 mit Dialkylformamid (Extraktionsmittel 1), das mit dem Lösungsmittel 1 eine Mischungslücke aufweist, in einer Flüssig-Flüssig-Extraktion.

2. Verfahren nach Anspruch 1, wobei für die Abtrennung der Ascorbinsäure aus dem Gemisch aus Ascorbinsäure und KGS das Extraktionsmittel N,N-Dibutylformamid ist.

3. Verfahren nach Anspruch 1 oder 2, umfassend den folgenden weiteren Schritt:
(b) Rückextrahieren der Ascorbinsäure aus dem beladenen Extraktionsmittel 1 mit einem polaren Extraktionsmittel 2, wobei ein mit Ascorbinsäure beladenes Extraktionsmittel 2 erhalten wird.

4. Verfahren nach einem der Ansprüche 1 bis 3, wobei das Lösungsmittel 1 und/oder das Extraktionsmittel 2 Wasser oder eine wässrige Lösung ist.

5. Verfahren nach Anspruch 3 oder 4, wobei die Extraktionstemperatur des Schrittes (a) T₁ 5°C bis 100°C niedriger ist als die Rückextraktionstemperatur des Schrittes (b) T₂.

6. Verfahren nach einem der Ansprüche 3 bis 5, umfassend den folgenden weiteren Schritt:
(c) Rückführen des Extraktionsmittels 1, aus dem die Ascorbinsäure gemäß Schritt (b) rückextrahiert wurde, in die Extraktion nach Schritt (a).

7. Verfahren nach einem der Ansprüchen 3 bis 6, umfassend folgende weiteren Schritte:
(d) Aufkonzentrieren des mit der Ascorbinsäure beladenen Extraktionsmittels 2; und
(e) optional, Rückführen des in (d) abgedampften Extraktionsmittels 2 (Brüden) in die Rückextraktion nach Schritt (b) als Extraktionsmittel 2.

8. Verfahren nach einem der Ansprüchen 3 bis 7, umfassend folgenden weiteren Schritt:
(f) Isolieren der Ascorbinsäure aus dem mit der Ascorbinsäure beladenen Extraktionsmittel 2, wobei eine Mutterlauge zurückbleibt.

9. Verfahren nach Anspruch 8, umfassend den folgenden weiteren Schritt:
(g) Extrahieren von Ascorbinsäure aus der bei einer Kristallisation der Ascorbinsäure gemäß Schritt (f) zurückbleibenden Mutterlauge.

10. Verfahren nach Anspruch 9, wobei zum Extrahieren der Ascorbinsäure aus der Mutterlauge die Mutterlauge der Extraktion nach Schritt (a) zugeführt wird.

11. Verfahren nach einem der Ansprüche 1 bis 10, wobei mit KGS beladenes Lösungsmittel 1 aus der Extraktion gemäß Schritt
(a) in ein Verfahren zur Herstellung von Ascorbinsäure aus KGS zurückgeführt wird.

## Claims

1. A process for removing ascorbic acid from a polar solvent 1 comprising ascorbic acid and 2-keto-L-gulonic acid, wherein the process comprises the following step:
(a) extracting the ascorbic acid from the solvent 1 with dialkylformamide (extraction medium 1), which has a miscibility gap with the solvent 1, in a liquid-liquid extraction.

2. The process according to claim 1, wherein, for removing the ascorbic acid from the mixture of ascorbic acid and KGA, the extraction medium is N,N-dibutylformamide.

3. The process according to claim 1 or 2 comprising the following further step:
(b) back-extracting the ascorbic acid from the loaded extraction medium 1 using a polar extraction medium 2, an extraction medium 2 loaded with ascorbic acid being obtained.

4. The process according to one of claims 1 to 3, wherein the solvent 1 and/or the extraction medium 2 is water or an aqueous solution.

5. The process according to claim 3 or 4, wherein the extraction temperature of the step (a) T₁ is from 5°C to 100°C lower than the back-extraction temperature of the step (b) T₂.

6. The process according to one of claims 3 to 5 comprising the following further step:
(c) recirculating the extraction medium 1 from which the ascorbic acid has been back-extracted as under step (b) to the extraction as under step (a).

7. The process according to one of claims 3 to 6 comprising the following further steps:
(d) concentrating the extraction medium 2 which is loaded with ascorbic acid; and
(e) optionally, recirculating the extraction medium 2 vaporized in (d) (vapors) to the back-extraction as under step (b) as extraction medium 2.

8. The process according to one of claims 3 to 7 comprising the following further step:
(f) isolating ascorbic acid from the extraction medium 2 loaded with ascorbic acid, a mother liquor remaining.

9. The process according to claim 8 comprising the following further step:
(g) extracting ascorbic acid from the mother liquor remaining in a crystallization of the ascorbic acid as under step (f).

10. The process according to claim 9, wherein, for the extraction of the ascorbic acid from the mother liquor, the mother liquor is fed to the extraction as under step (a).

11. The process according to one of claims 1 to 10, wherein KGA-loaded solvent 1 from the extraction as under step (a) is recirculated to a process for preparing ascorbic acid from KGA.

## Revendications

1. Procédé de séparation d'acide ascorbique d'un solvant polaire 1 contenant de l'acide ascorbique et de l'acide 2-céto-L-gulonique (KGS), **caractérisé en ce que** le procédé comprend l'étape suivante :
(a) l'extraction de l'acide ascorbique du solvant 1 avec du dialkylformamide (agent d'extraction 1), qui présente avec le solvant 1 une lacune de miscibilité, dans une extraction liquide-liquide.

2. Procédé selon la revendication 1, dans lequel l'agent d'extraction pour la séparation de l'acide ascorbique du mélange d'acide ascorbique et de KGS est le N,N-dibutylformamide.

3. Procédé selon la revendication 1 ou 2, comprenant l'autre étape suivante :
(b) la réextraction de l'acide ascorbique de l'agent d'extraction chargé 1 avec un agent d'extraction polaire 2, pour obtenir un agent d'extraction 2 chargé d'acide ascorbique.

4. Procédé selon l'une quelconque des revendications 1 à 3, dans lequel le solvant 1 et/ou l'agent d'extraction 2 est de l'eau ou une solution aqueuse.

5. Procédé selon la revendication 3 ou 4, dans lequel la température d'extraction de l'étape (a) T₁ est de 5°C à 100°C plus basse que la température de réextraction de l'étape (b) T₂.

6. Procédé selon l'une quelconque des revendications 3 à 5, comprenant l'autre étape suivante :
(c) le recyclage de l'agent d'extraction 1 dont on a réextrait l'acide ascorbique selon l'étape (b), dans l'extraction selon l'étape (a).

7. Procédé selon l'une quelconque des revendications 3 à 6, comprenant les autres étapes suivantes :
(d) la reconcentration de l'agent d'extraction 2 chargé d'acide ascorbique; et
(e) éventuellement, le recyclage de l'agent d'extraction 2 vaporisé dans (d) (fumées) dans la réextraction selon l'étape (b) comme agent d'extraction 2.

8. Procédé selon l'une quelconque des revendications 3 à 7, comprenant l'autre étape suivante :
(f) l'isolement de l'acide ascorbique de l'agent d'extraction 2 chargé de l'acide ascorbique, dans lequel reste une lessive mère.

9. Procédé selon la revendication 8, comprenant l'autre étape suivante :
(g) l'extraction d'acide ascorbique de la lessive mère restant lors d'une cristallisation de l'acide ascorbique selon l'étape (f).

10. Procédé selon la revendication 9, dans lequel, pour extraire l'acide ascorbique de la lessive mère, on achemine la lessive mère à l'extraction selon l'étape (a).

11. Procédé selon l'une quelconque des revendications 1 à 10, dans lequel on recycle le solvant 1 chargé de KGS de l'extraction selon l'étape (a) dans un procédé de fabrication d'acide ascorbique à partir de KGS.
